(19) Europäisches Patentamt
European Patent Office
Office européen des brevets

(11) Veröffentlichungsnummer: **0 271 023**
**A2**

(12) # EUROPÄISCHE PATENTANMELDUNG

(21) Anmeldenummer: **87117972.7**

(22) Anmeldetag: **04.12.87**

(51) Int. Cl.⁴: **C07C 101/28**, C07C 103/30, C07C 103/133, A61K 31/16, A61K 31/195

(30) Priorität: **06.12.86 DE 3641822**

(43) Veröffentlichungstag der Anmeldung: **15.06.88 Patentblatt 88/24**

(84) Benannte Vertragsstaaten: **AT BE CH DE ES FR GB GR IT LI LU NL SE**

(71) Anmelder: **GÖDECKE AKTIENGESELLSCHAFT**
**Salzufer 16**
**D-1000 Berlin 10(DE)**

(72) Erfinder: **Weiershausen, Ute**
**Bundesstrasse 70**
**D-7803 Gundelfingen(DE)**
Erfinder: **Satzinger, Gerhard, Dr.**
**Im Mattenbühl 7**
**D-7809 Denzlingen(DE)**
Erfinder: **Geibel, Wolfram, Dr.**
**Schillerstrasse 23**
**D-6418 Hünfeld(DE)**

(54) **Verwendung von Dihydrophenylaminosäurederivaten und diese enthaltende Arzneimittel zur Immunmodulation und Cytostase.**

(57) Die Erfindung betrifft eine neue Klasse spezifisch wirkender Immunsuppressiva der allgemeinen Formel I

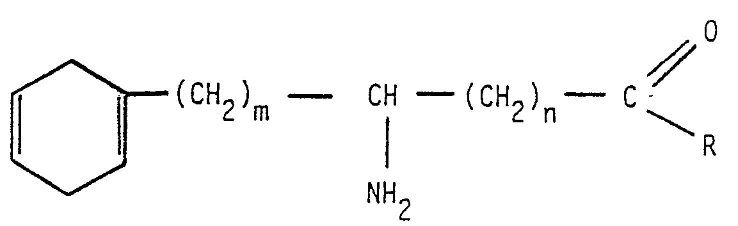

(I)

in welcher R eine gegebenenfalls substituierte Amino-oder Hydroxylgruppe darstellt und m und n unabhängig voneinander die Zahlen 0 oder 1 bedeuten, sowie deren pharmakologisch unbedenkliche Salze.

Die Verbindungen der Formel I hemmen T-Lymphozyten spezifisch bei niedriger Toxizität und wirken in hohen Dosen cytostatisch.

EP 0 271 023 A2

# BESCHREIBUNG

Die derzeit verwendeten klassischen Immunsuppressiva und meisten gebräuchlichen Cytostatika haben eine gravierende Nebenwirkung gemeinsam, die dosislimitierend und oft lebensbedrohlich ist: Sie sind knochenmarkstoxisch und führen zu einer quantitativen Reduktion der weißen Blutzellen in der Zirkulation. Diese Zellpopulation enthält in der Lymphozytenfraktion sogenannte T- und B-Lymphozyten. Eine Subpopulation des erstgenannten Zelltyps, die "zytotoxischen T-Lymphozyten", sind maßgeblich an der Abstoßung transplantierter Gewebe nicht verwandter Spender beteiligt. B-Lymphozyten dagegen sorgen insbesondere für die Abwehr bakterieller Infekte. Demnach verhindern konventionelle Immunsuppressiva bei Transplantatempfängern die Abstoßung der übertragenen Organe durch Verminderung der peripheren T-Lymphozyten. Da die unspezifischen Wirkstoffe jedoch auch B-Lymphozyten reduzieren, können Patienten unter dieser Medikation durch eine gesteigerte Anfälligkeit gegenüber bakteriellen Infekten vital gefährdet sein. Dies ist ebenso der Fall bei Patienten, die aufgrund eines malignen Tumors oder einer benignen proliferativen Erkrankung (z.B. Psoriasis, Arthritis) mit einem Cytostatikum behandelt werden. Die Verminderung der weißen Blutzellen durch konventionelle Immunsuppressiva und Cytostatika kommt dadurch zustande, daß von ihrer zelltoxischen Wirkung die schnell proliferierenden Gewebe, also auch das hämatopoetische System, bevorzugt betroffen sind.

Aufgabe der Erfindung ist die Bereitstellung von Immunsuppressiva, welche zytotoxische T-Lymphozyten nicht durch Dezimierung sondern durch spezifische Hemmung ihrer immunologischen Funktionen beeinflussen.

Cyclosporin, interferiert zwar mit der Bildung von Interleukin-2, einem von Helfer-T-Lymphozyten zur Aktivierung der zytotoxischen T-Lymphozyten produzierten Botenstoff. Wenn die Aktivierung der Zellen ausbleibt, kann ein übertragenes

Fremdtransplantat nicht abgestoßen werden. Cyclosporin ist daher aufgrund seiner relativen Spezifität bereits als Fortschritt gegenüber den konventionellen immunsuppressiven Standards (Azathioprin, Corticosteroide) zu betrachten. Es mehren sich aber in neuerer Zeit Berichte über teils schwerwiegende Nebenwirkungen dieser Substanz. Cyclosporin ist nierentoxisch infolge Mikroembolisation und kann Wucherungen der Mundschleimhaut, multiple kutane Reaktionen, ZNS Wirkungen wie Tremor u.a. auslösen.

Es wurde nun überraschend gefunden, daß Dihydrophenylaminosäurederivate der allgemeinen Formel I

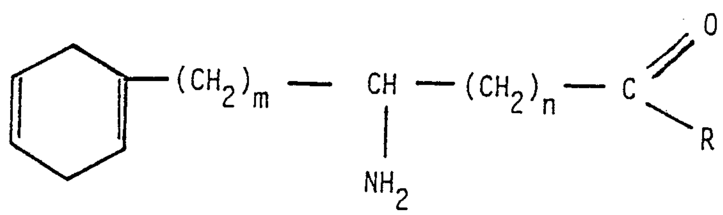

(I)

in welcher R eine gegebenenfalls substituierte Amino- oder Hydroxylgruppe darstellt und m und n unabhängig voneinander die Zahlen 0 oder 1 bedeuten, sowie deren pharmakologisch unbedenkliche Salze

die immunologischen Funktionen der T-Lymphozyten spezifisch hemmen und aufgrund ihrer niedrigen Toxizität auch in hoher Dosierung hervorragend verträglich sind.

Der Substituent R in der Formel (I) scheint für die Wirkung der neuen Verbindungen wenig auschlaggebend zu sein. Nach derzeitigem Kenntnisstand ist das Wirkprinzip mit großer Wahrscheinlichkeit vor allem an das Vorhandensein eines mit einer Aminosäuregruppierung verbundenen Dihydrophenylrestes gebunden.

Der Rest R kann somit praktisch jeder untoxische organische Rest sein, insbesondere kommen unsubstituierte und substituierte Amino- und Hydroxygruppen und als deren Substituenten gesättigte und ungesättigte niedere aliphatische oder cycloaliphatische Reste mit 1 bis 8, bevorzugt 1-4 Kohlenstoffatomen, wie z.B. der Methyl-, Ethyl-, Propyl-, Isopropyl-, Cyclopentyl-, Cyclohexyl-, Allyl- oder Butenylrest infrage.

Ein Vertreter mit relativ großem Rest R ist z.B. das Cefradin (m = o, N = o, R = 7-Amino-3-deacetoxycephalosporansäure), welches seit Jahren zur Behandlung bakterieller Infekte im Handel ist. Die Substanz wird klinisch außerordentlich gut vertragen und zeigt im Tierversuch selbst bei länger dauernder hochdosierter Anwendung keine Nierentoxizität, was keineswegs für alle Cephalosporine zutrifft. Insbesondere wirkt Cefradin nicht leukopenisch. Es ist daher überraschend, daß Cefradin die Abstoßung allogener Transplantate unterdrückt. Der Nachweis dieser Aktivität konnte überraschend im Rattenhaut-Transplantationsmodell erbracht werden (s. Tab. I). Als bekanntes Arzneimittel eignet sich Cefradin sehr gut als Modellsubstanz für den Nachweis der neuartigen Wirksamkeit der Verbindungen I.

In diesem Testsystem nach Waynforth (Experimental and Surgical Technique in the Rat, Academic Press, 1980) modifiziert nach Heystek, werden je 2 allogene Hauttransplantate histoinkompatibler Spender-Ratten auf Empfänger-Ratten übertragen.

Die mit einer Stanze gewonnenen und vom Panniculus carnosus (einer Fett- und Muskelschicht) präparativ befreiten Transplantate werden in ebenfalls ausgestanzte Transplantatbetten des Empfängertiers plaziert. Die Transplantate werden mit speziellem Verbandmaterial bedeckt und mit einer Bandage fixiert. Endpunkt der Untersuchung ist der Tag, an dem 50 % der Tiere beide Transplantate vollständig abgestoßen haben. Die immunsuppressive Aktivität eines Wirkstoffs wird anhand der Verlängerung des normalen

Transplantatüberlebens (Tage) der nicht behandelten Kontrolltiere bestimmt.

Wie Tab. I zu entnehmen, zeigt sich Cefradin in dem beschriebenen Modell außerordentlich wirksam und dem immunsuppressiven Standard Azathioprin {6[(1-Methylnitroimidazol-5-yl)thio]purin} deutlich überlegen. Besonders interessant ist dabei der Befund, daß Azathioprin in äquipotenter Dosis ausgeprägt toxisch wirkt, während die mit Cefradin behandelten Tiere keinerlei Unverträglichkeit erkennen ließen. Interessant war weiterhin der Befund, daß die höchste Cefradin Dosis offenbar die Ausbildung der Gefäßverbindung zwischen Transplantatbett und Transplantat unterdrückte, die bei den Kontrolltieren bis etwa zum 5. postoperativen Tag hergestellt ist.

Aufgrund dieser Beobachtung wurde zunächst angenommen, daß eine Unterdrückung der Angiogenese durch die erfindungsgemäßen Substanzen für die Verlängerung des Transplantatüberlebens in dem verwendeten Modell verantwortlich ist. Wenn eine Gefäßverbindung zwischen Transplantatbett und Transplantat nicht besteht, kann nämlich eine Sensibilisierung zytotoxischer T-Lymphozyten durch die übertragenen Fremdantigene und Einwanderung dieser Zellen in das Transplantat, welche die Abstoßung zur Folge hätte, nicht stattfinden.

Es wurde daher eine Untersuchung mit Cefradin als Modellsubstanz auf eine die Gefäßbildung hemmende Aktivität in einem hierzu geeigneten in vitro Modell durchgeführt (s. Tab. II). In diesem Test wird der Einfluß auf die Motilität boviner Endothelzellen unter angiogenem (gefäßbildendem) Stimulus bestimmt. Es wurde gefunden, daß Cefradin in einem Konzentrationsbereich, der den in vivo eingesetzten niedrigen, jedoch bereits ausgeprägt immunsuppressiven Dosen entsprach, eine nur geringfügige, wenn auch dosisabhängige Aktivität zeigte. In hoher, bereits zytotoxischer Konzentration wurde unter den gegebenen Versuchsbedingungen eine komplette Inhibition der Endothelmotilität bewirkt.

Aufgrund der dargelegten Befunde muß ausgeschlossen werden, daß die durch die erfindungsgemäßen Substanzen in niedriger Dosierung bewirkte ausgeprägte Verlängerung des Transplantatüberlebens über konventionelle immunsuppressive oder antiangiogene Mechanismen zustande kommt. Statt dessen ist eine Modulation von für die Abstoßungsreaktion wesentlichen Funktionen zytotoxischer T-Lymphozyten oder der diese kontrollierenden Zellpolulationen anzunehmen.

Die Inhibition des Gefäßwachstums durch sehr hohe Dosen der erfindungsgemäßen Substanz ist wie die immunmodulierende Aktivität von besonderem therapeutischem Interesse.

Nach derzeitigem Wissensstand ist eine pathologisch gesteigerte Angiogenese an verschiedenen proliferativen Erkrankungen wie soliden Tumoren, Psoriasis, Arthritis, chronischer Inflammation und diabetischer Retinopathie beteiligt, deren Regulierung zu einer Besserung wenn nicht Heilung der betreffenden Erkrankungen führen kann. Angiogenese-Inhibitoren stellen daher nach allgemeiner Auffassung ein erfolgversprechendes Therapiekonzept für die genannten, mit konventionellen Arzneimitteln bisher kaum zu beherrschenden proliferativen Erkrankungen dar.

Daß der Dihydrophenylrest eine für die Wirksamkeit der Verbindungen I wichtige Funktion hat ergibt sich aus einem Vergleich von Cefradin mit Cefradoxil, deren Formeln nachstehend einander gegenübergestellt sind:

Cefradin
(aktiv)

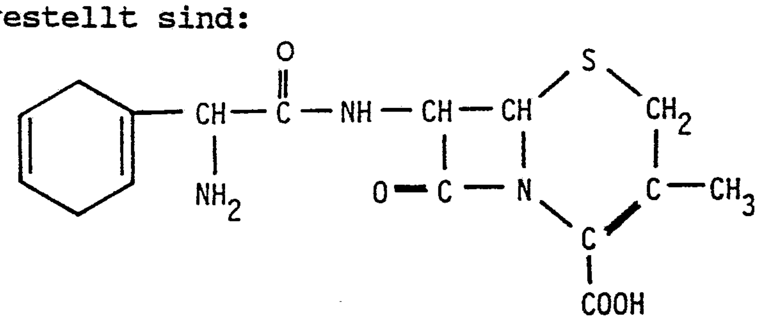

Cefradoxil
(inaktiv)

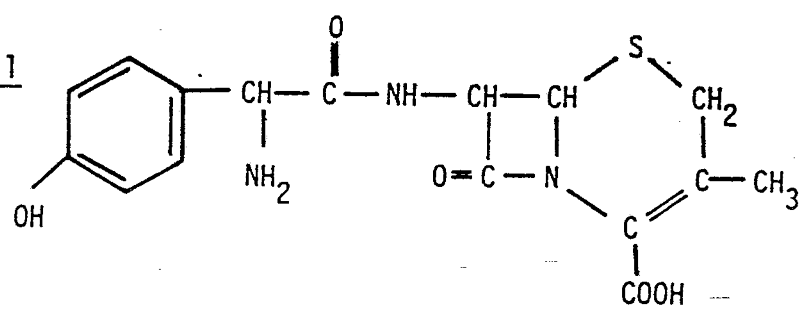

Es kommt also nicht auf das Cephalosporin-Grundgerüst an, sondern auf die für die erfindungsgemäßen Verbindungen typische, eine Dihydrophenylgruppierung enthaltende Teilstruktur, welche für die immunmodulierende Aktivität ausschlaggebend ist.

Weitere an sich bekannte Verbindungen der allgemeinen Formel I sind z.B. Dihydrophenylglycin, Dihydrophenylalanin und Dihydrophenyl-alaninamid.

Neu, aber nach den für die obengenannten Verbindungen allgemein bekannten Verfahren herstellbar sind folgende Verbindungen der allgemeinen Formel I:

Dihydrophenylglycinamid
Dihydrophenyl-ß-alaninamid
Dihydrophenylglycin-N-methylamid
Dihydrophenylglycin-N-ethylamid
Dihydrophenylglycin-N-isopropylamid
Dihydrophenyl-ß-alanin-N-methylamid
Dihydrophenyl-ß-alanin-N-ethylamid
Dihydrophenyl-ß-alanin-N-isopropylamid

Für die neuen Verbindungen wird Stoffschutz beansprucht.

Die Salze werden in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen

Zusätze wie Stabilisierungsmittel, Lösungsvermittler oder Puffer enthält.

Derartige Zusätze sind z.B. Tartrat- und Citrat-Puffer, Ethanol, Komplexbildner (wie Äthylendiamin-tetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäure, höhermolekulare Fettsäure (wie Stearinsäure), Gelatine, Agar-Agar, Calciumphosphat, Natriumcarbonat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Die enteral verabreichten Einzeldosen liegen im Bereich von etwa 10 bis 6000 mg, vorzugsweise 50-500 mg, parenteral werden etwa 1 bis 8000 mg gegeben.

Gegenstand der vorliegenden Erfindung sind somit Arzneimittel, enthaltend eine Verbindung der allgemeinen Formel I und deren pharmakologisch unbedenkliche Salze, mit Ausnahme von Cefradin, als Wirkstoff neben üblichen Träger und Hilfsstoffen.

Ein weiterer Gegenstand der Erfindung ist die Verwendung von Verbindungen der allgemeinen Formel I, insbesondere Cefradin, und von deren pharmakologisch unbedenklichen Salzen bei der Immunmodulation und Cytostase.

Die Wirkstoffe gemäß Formel I enthaltenden Arzneimittel begründen ein hochverträgliches neuartiges therapeutisches Prinzip für Störungen des Immunsystems und Erkrankungen, bei denen eine gesteigerte benigne oder maligne Zellproliferation und/oder erhöhte enzymatische und angiogene Aktivitäten beteiligt sind. Hierzu zählen u.a. rheumatische Erkrankungen, Arthritis, Autoimmunerkrankungen, Psoriasis, maligne Tumoren, bestimmte Erkrankungen der Atemwege sowie die diabetische

Retinopathie. Die erfindungsgemäßen Substanzen eignen sich weiterhin zur Unterdrückung der Abstoßung histoinkompatibler Transplantate.

Aus der folgenden Tabelle I geht hervor, daß Cefradin als wirksamer Vertreter der neuen Wirkstoffklasse dem Azathioprin überlegen ist. Außerdem zeigt Tabelle II die Unwirksamkeit von Cefradoxil. Tabelle III zeigt den Einfluß von Cefradin auf die in vitro Migration/Motilität boviner Endothelzellen.

## T A B E L L E I

Immunsuppressive Aktivität von Cefradin in nicht-toxischer Dosierung von 250, 500 bzw. 1000 mg/kg/Tag.
Positivstandard: Azathioprin in maximal tolerierter Dosierung von 40 mg/kg/Tag.

Modell: Hauttransplantat-Überlebenstest, Ratte
Empfänger: BDE ♂, Spender Lewis ♂

Es wird der Tag bestimmt, an dem mindestens 50% der Tiere einer Gruppe beide Transplantate abgestoßen haben.

| Prüfsubstanz Dosierung | Tiere mit 2 abgestoßenen Transplantaten / n (%) | | | | | | | | | | | | |
|---|---|---|---|---|---|---|---|---|---|---|---|---|---|
| | Tag +10 | 11 | 12 | 13 | 14 | 15 | 16 | 17 | 18 | 19 | 20 | 21 | 22 |
| Kontrolle Methocel i.g. ab Tag - 2 bis zur Abstoßg. | 1/7 (14) | 1/7 (14) | 1/7 (14) | 4/7 (57) | 7/7 (100) | | | | | | | | |
| Cefradin 2xtägl. 125mg/kg i.g. ab Tag -2 bis zur Abstoßung | 0/8 (0) | 0/8 (0) | 0/8 (0) | 1/8 (13) | 2/8 (25) | 4/8 (50) | 7/8 (88) | 7/8 (88) | 8/8 (100) | | | | |
| Cefradin 2xtägl. 250mg/kg i.g. ab Tag -2 bis zur Abstoßung | 0/7 (0) | 0/7 (0) | 0/7 (0) | 0/7 (0) | 1/7 (14) | 5/7 (71) | 6/7 (86) | 6/7 (86) | 6/7 (86) | 6/7 (86) | 6/7 (86) | 6/7 (86) | 7/7 (100) |
| Cefradin 2xtägl. 500mg/kg i.g. ab Tag -2 bis zur Abstoßung | 0/8 (0) | 1/8 (13) | 1/8 (13) | 1/8 (13) | 1/8 (13) | 2/8 (25) | 2/8 (25) | 3/8 (38) | 5/8 (63) | 5/8 (63) | 6/8 (75) | 8/8 (100) | |
| Azathioprin 2xtägl. 20mg/kg i.g. ab Tag -2 bis zur Abstoßung | 0/8 (0) | 0/8 (0) | 1/8 (13) | 1/8 (13) | 3/8 (38) | 7/8 (88) | 8/8 (100) | | | | | | |

# T A B E L L E  II

Untersuchung der Wirkung von Cefradin auf die in vitro Migration/Motilität boviner Endothelzellen unter angiogenem Stimulus.

Methode: Boyden-Kammer, modifiziert durch Präinkubation der Zellen mit Gangliosid-Mischung (Gullino P.M., Invasion and Metastatis 6: 145 - 165, 1986).

Es wird die Anzahl der Zellen bestimmt, die innerhalb 8 Stunden durch den mit Kollagen bzw. Kollagen + Fibronektin belegten Filter passieren.

| Präinkubation * | Cefradin µg/ml Medium | Anzahl der migrierten Zellen** | |
|---|---|---|---|
| | | Kollagen-Filter | Kollagen-Fibronektin-Filter |
| - | 0 | 23 ± 3 | 87 ± 12 |
| + | 0 | 162 ± 34 | 359 ± 16 |
| + | 10 | 173 ± 29 | 306 ± 74 |
| + | 100 | 104 ± 3 | 256 ± 24 |
| + | 1000 | 23 ± 8 | 51 ± 6 |

*Gangliosid-Mischung: $GM_1$ = 50%, $GD_1$ = 35%, $GT_1$ = 15%

**Mittelwert (± Standardabweichung) von 3 Versuchen je Gruppe und Filter

# TABELLE III

Immunsuppressive Aktivität von Cefradin und Cefadroxil. Positivstandard: Azathioprin.

Modell: Hauttransplantat-Überlebenstest, Ratte

Empfänger: BDE ♂, Spender: Lewis ♂

Es wird der Tag bestimmt, an dem mindestens 50% der Tiere einer Gruppe beide Transplantate abgestoßen haben.

| Prüfsubstanz / Dosierung | Tiere mit 2 abgestoßenen Transplantaten / n (%) | | | | | | | | | | | | |
|---|---|---|---|---|---|---|---|---|---|---|---|---|---|
| | Tag + 9 | 10 | 11 | 12 | 13 | 14 | 15 | 16 | 17 | 18 | 19 | 20 | 21 |
| Kontrolle<br>Methocel i.g. ab Tag - 2 bis zur Abstoßg. | 0/6 (0) | 0/6 (0) | 1/6 (17) | 3/6 (50) | 4/6 (67) | 6/6 (100) | | | | | | | |
| Azathioprin*<br>50mg/kg i.g. ab Tag - 2 bis Tag + 6 | 0/4 (0) | 0/4 (0) | 0/4 (0) | 0/4 (0) | 1/4 (25) | 2/4 (50) | 2/4 (50) | 4/4 (100) | | | | | |
| Cefradin<br>100mg/kg i.g. ab Tag - 2 bis zur Abstoßg. | 0/6 (0) | 0/6 (0) | 0/6 (0) | 0/6 (0) | 1/6 (17) | 3/6 (50) | 4/6 (67) | 5/6 (83) | 5/6 (83) | 5/6 (83) | 5/6 (83) | 6/6 (100) | |
| Cefadroxil<br>100mg/kg i.g. ab Tag - 2 bis zur Abstoßg. | 0/6 (0) | 0/6 (0) | 1/6 (17) | 3/6 (50) | 4/6 (67) | 4/6 (67) | 4/6 (67) | 5/6 (83) | 6/6 (100) | | | | |

*Azathioprin mußte wegen Toxizität (2 Tiere ex.) vorzeitig abgesetzt werden.

PATENTANSPRÜCHE (für die Vertragsstaaten AT, BE, CH, DE, FR, GB, IT, LI, LU, NE, SE)

1.) Arzneimittel, enthaltend eine Verbindung der allgemeinen Formel I

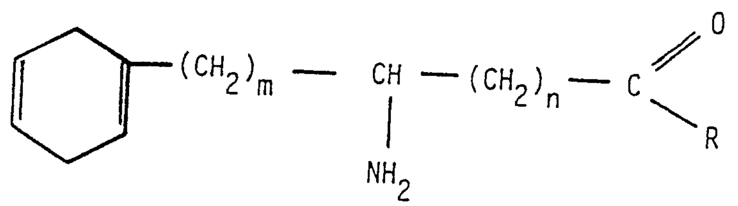

(I)

in welcher R eine gegebenenfalls substituierte Amino- oder Hydroxylgruppe darstellt und m und n unabhängig voneinander die Zahlen 0 oder 1 bedeuten, und deren pharmakologisch unbedenkliche Salze, mit Ausnahme von Cefradin, als Wirkstoff neben üblichen Trägern und Hilfsstoffen.

2.) Verwendung von Verbindungen der allgemeinen Formel I in welcher R eine gegebenenfalls substituierte Amino- oder Hydroxylgruppe darstellt und m und n unabhängig voneinander die Zahlen 0 oder 1 bedeuten, sowie deren pharmakologisch unbedenkliche Salze

und von deren pharmakologisch unbedenklichen Salzen zur Herstellung von Arzneimitteln zur Immunmodulation und Cytostase.

3.) Verwendung eines Wirkstoffs nach Anspruch 2 zur Herstellung von Arzneimitteln zur Bekämpfung von Autoimmunerkrankungen, rheumatischen und allergischen Erkrankungen, benignen und malignen proliferativen Erkrankungen und zur Unterdrückung der Transplantatabstoßung.

4.) Verbindungen der allgemeinen Formel I und deren pharmakologisch verträgliche Salze nämlich Dihydrophenylglycinamid,

Dihydrophenyl-ß-alaninamid,
Dihydrophenylglycin-N-methylamid,
Dihydrophenylglycin-N-ethylamid,
Dihydrophenylglycin-N-isopropylamid,
Dihydrophenyl-ß-alanin-N-methylamid,
Dihydrophenyl-ß-alanin-N-ethylamid,
Dihydrophenyl-ß-alanin-N-isopropylamid.

5.) Arzneimittel enthaltend eine Verbindung gemäß Anspruch 4.

6.) Verwendung von Verbindungen gemäß Anspruch 4 zur Herstellung von Arzneimitteln zur Immunmodulation, Cytostase, und Bekämpfung von Autoimmunerkrankungen, rheumatischen und allergischen Erkrankungen, benignen und malignen proliferativen Erkrankungen, und zur Unterdrückung der Transplantatabstoßung.

PATENTANSPRÜCHE (für die Vertragsstaaten ES, GR)

1.) Verwendung von Verbindungen der allgemeinen Formel I

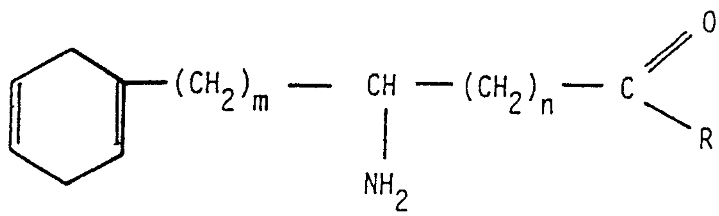

(I)

in welcher R eine gegebenenfalls substituierte Amino- oder Hydroxylgruppe darstellt und m und n unabhängig voneinander die Zahlen 0 oder 1 bedeuten, und deren pharmakologisch unbedenkliche Salze, mit Ausnahme von Cefradin, als Wirkstoff neben üblichen Trägern und Hilfsstoffen zur Herstellung von Arzneimitteln.

2.) Verwendung von Verbindungen der allgemeinen Formel I in welcher R eine gegebenenfalls substituierte Amino- oder Hydroxylgruppe darstellt und m und n unabhängig voneinander die Zahlen 0 oder 1 bedeuten, sowie deren pharmakologisch unbedenkliche Salze

und von deren pharmakologisch unbedenklichen Salzen zur Herstellung von Arzneimitteln zur Immunmodulation und Cytostase.

3.) Verwendung eines Wirkstoffs nach Anspruch 2 zur Herstellung von Arzneimitteln zur Bekämpfung von Autoimmunerkrankungen, rheumatischen und allergischen Erkrankungen, benignen und malignen proliferativen Erkrankungen und zur Unterdrückung der Transplantatabstoßung.

4.) Verfahren zur Herstellung von Verbindungen der allgemeinen Formel I und deren pharmakologisch verträgliche Salzen nämlich

Dihydrophenylglycinamid,

Dihydrophenyl-ß-alaninamid,

Dihydrophenylglycin-N-methylamid,
Dihydrophenylglycin-N-ethylamid,
Dihydrophenylglycin-N-isopropylamid,
Dihydrophenyl-ß-alanin-N-methylamid,
Dihydrophenyl-ß-alanin-N-ethylamid,
Dihydrophenyl-ß-alanin-N-isopropylamid.

5.) Verwendung von Verbindungen gemäß Anspruch 4 zur Herstellung von Arzneimitteln zur Immunmodulation, Cytostase, und Bekämpfung von Autoimmunerkrankungen, rheumatischen und allergischen Erkrankungen, benignen und malignen proliferativen Erkrankungen, und zur Unterdrückung der Transplantatabstoßung.